# EUROPÄISCHE PATENTANMELDUNG

(11) **EP 1 698 279 A1**
(43) Veröffentlichungstag der Anmeldung: **06.09.2006**
(21) Anmeldenummer: 05004760.4
(22) Anmeldetag: 04.03.2005
(51) Int. Cl.: A61B 5/15

(54) **Sequentielle Einführung von Hauptpenetratoren**

(71) Anmelder: Disetronic Licensing AG, 3401 Burgdorf (CH)
(72) Erfinder: Haueter, Ulrich, CH-3506 Grosshöchstetten (CH); Niederhäuser, Sandro, CH-3368 Bleienbach (CH); Feldmann, Peter, 3326 Krauchthal (CH); Hof, Christian, CH-3007 Bern (CH)
(74) Vertreter: Küng, Peter

(57) **Zusammenfassung**

Die vorliegende Erfindung betrifft eine Vorrichtung zum Setzen von Hautpenetratoren (1) mit einem Stechteil (5), das die Haut durchsticht, und mit einem Verweilteil (11), der durch eine vom Stechteil (5) erzeugte Öffnung in die Haut eingebracht wird und dort verbleibt. Die erfindungsgemässe Setzvorrichtung ist dadurch gekennzeichnet, dass das Stechteil (5) und Verweilteil (11) separat an der Setzvorrichtung (1) vorgesehen sind, und durch mindestens eine Führung (12, 24, 35, 40) in oder an welcher das Verweilteil (11) nach dem Stechteil (5) in dessen Stechposition verbracht wird, um dort in die Haut gesetzt zu werden oder der Stechteil (5) und der Verweilteil (11) sind als separate Vorrichtungen ausgestaltet.

## Beschreibung

Die vorliegende Erfindung betrifft das sequentielle Setzen von Hautpenetratoren. Mit dem Begriff "Hautpenetratoren" sind im Kontext des vorliegenden Dokuments Vorrichtungen gemeint, die von außen durch die Haut in den Körper eingebracht werden. Es können z.B. Kanülen zur Verabreichung von Medikamenten sein, aber auch Sonden, die in die Haut oder das darunter liegende Gewebe eingestochen werden sowie Sensoren zur Messung eines physiologischen Parameters wie z.B. Glucosesensoren.

Als wesentliche Elemente weisen solche Vorrichtungen ein Stechteil auf, das die Haut durchsticht, sowie ein Verweilteil, das in eine vom Stechteil erzeugte Hautöffnung bzw. in einen Hautkanal eingebracht wird und dort verbleibt. Bekannte Ausführungen haben eine relativ weiche, aus Kunststoffmaterial hergestellte Kanüle, die das Verweilteil bildet, sowie eine mit einer Spitze versehene Stahlkanüle, welche durch die Kunststoffkanüle hindurch verläuft, wobei die Spitze vorne aus der Kunststoffkanüle herausragt. Beim Setzen dieser bekannten Ausführungsformen werden Stechteil und Verweilteil gemeinsam eingestochen, wonach die Stahlkanüle aus der Kunststoffkanüle herausgezogen wird, so dass nur die Kunststoffkanüle im Körper verbleibt. Die Kunststoffkanüle ist an ein Leitungssystem angeschlossen (meist über einen Adapter, der auch als Führung für die Stahlkanüle dienen kann) um so Flüssigkeit durch die Haut ein- oder auszubringen.

Die Nachteile solcher bekannter Systeme sind insbesondere die Folgenden: Die Stahlkanüle muss in einem separaten Bedienschritt entfernt und entsorgt werden. Wenn sie während der Tragedauer im Körper verbleibt, wird dies als störend oder als schmerzhaft empfunden. Die Schliffgeometrie für die Spitze der Stahlkanüle muss über die Kunststoffkanüle hinausragen und verlängert so die Gesamtlänge um mehrere Millimeter. Die Einbringung der Stahlkanüle in die Kunststoffkanüle bei der Herstellung des Systems gestaltet sich schwierig, wenn die weiche Kunststoffkanüle auch nur ein wenig abgebogen ist. Die Kunststoffkanüle könnte dann, durch die Spitze der Stahlkanule verletzt oder zerstört werden.

Es ist die Aufgabe der vorliegenden Erfindung, eine Vorrichtung zum Setzen von Hautpenetratoren bereitzustellen, welche mindestens einen, vorzugsweise alle oben genannten Nachteile der bekannten Ausführungsformen überwindet. Insbesondere soll eine einfach handhabbare bzw. gut verträgliche und/oder einwandfrei funktionierende Setzvorrichtung für Hautpenetratoren bereitgestellt werden.

Diese Aufgabe wird durch eine Vorrichtung zum Setzen von Hautpenetratoren gemäß dem Anspruch 1 sowie durch ein Verfahren gemäß dem Anspruch 16 gelöst. Die Unteransprüche beschreiben bevorzugte Ausführungsformen der Erfindung.

Die Vorrichtung zum Setzen von Hautpenetratoren gemäß der vorliegenden Erfindung ist dadurch gekennzeichnet, dass das Stechteil und das Verweilteil separat an der Setzvorrichtung vorgesehen sind, sowie durch mindestens eine Führung in oder an welcher das Verbleibteil nach dem Stechteil in dessen Stechposition verbracht wird, um dort in die Haut gesetzt zu werden.
Alternativ können der Stechteil und das Verweilteil auch an zwei separaten Vorrichtungen angebracht sein oder als zwei separate Vorrichtungen ausgestaltet sein. Bevorzugt ist eine einteilige Ausgestaltung der Vorrichtung d.h. der Stechteil und der Verweilteil sind Teile einer Vorrichtung.

Mit anderen Worten werden Stechteil und Verbleibteil räumlich getrennt und nebeneinander bereitgestellt und nacheinander in die Haut eingebracht. Die Führung sorgt dafür, dass das Verweilteil genau in einer Position über dem vorgestochenen Loch ist, wenn es in die Haut eingebracht wird. Bevorzugt ist eine Vorrichtung, bei welcher das Stechteil und der Verweilteil in oder an einem Gehäuse angeordnet sind.

Vorteilhafterweise ist es damit nicht länger notwendig, das Stechteil länger zu machen als das Verweilteil. Beide Teile können gleich lang sein, so dass die Haut nicht weiter als notwendig penetriert wird. Bevorzugt ist ein Stechteil, der weniger lang ist als der Verweilteil, bevorzugter weniger als halb solang wie der Stechteil, noch bevorzugter weist der Stechteil eine Länge auf die weniger als ein Drittel der Länge des Verweilteils ausmacht.

Als mechanisches Stechteil kann z.B. eine Stahlnadel verwendet werden. Bevorzugt sind Stechnadeln, welche nicht hohl sind.

Der Anwender wird durch die Bereitstellung der Führung derart unterstützt, dass er unmöglich einen Fehler beim Einbringen des Verweilteils machen kann, sondern immer zuverlässig in den vorgestochenen Kanal trifft. Das Stechteil verbleibt nicht in dem Kanal, so dass Störungsgefühle beim Anwender ausbleiben.

Gemäß einer Ausführungsform der Erfindung kann die Führung den Stechteil und den Verweilteil führen, und sie kann einen Haltepunkt aufweisen, welcher der Stech- bzw. Setzposition zugeordnet ist. Der Stechteil kann z.B. einen Stechteilträger umfassen, an welchem ein Stechelement zur Eröffnung der Haut angebracht ist.

Dabei ist es möglich, den Haltepunkt durch einen Anschlag für den Stechteil und/oder den Verweilteil zu bilden. Ein solcher Anschlag hat immer den Vorteil, dass er eine größtmögliche Positionssicherheit gewährleistet. Wenn ein Anschlag vorgesehen wird, ist es von Vorteil, dass der Stechteil und der Verweilteil bewegungsgekoppelt in der Führung geführt werden, und der Haltepunkt durch einen solchen Anschlag erzeugt wird, an den der Stechteil nach dem Stechen herangeführt wird, wobei er den Verweilteil in die Setzposition mitnimmt.

Der oben schon angesprochene Haltepunkt kann grundsätzlich auch durch einen Rasteingriff für den Stechteil und/oder den Verweilteil gebildet werden, wobei der Rasteingriff in oder an der Führung angeordnet ist. In diesem Fall ist es von Vorteil, wenn ein einziger, wieder entrastbarer Rasteingriff in oder an der Führung vorgesehen ist, in dem zunächst der Stechteil und danach der Verweilteil einrasten kann.

Gemäß einer besonders bevorzugten Ausführungsform der Erfindung wird in oder an der Führung ein Rückhalteglied vorgesehen, welche das Stechteil in der ausgerückten Position hält (nach dem Vorstechen) oder die gekoppelte Einheit aus separatem Stechteil und Verweilteil in der Setzposition des Verweilteils hält. Der Vorteil liegt darin, dass nach dem Verbringen des Verweilteils in die Setzposition keine unbeabsichtigte Verschiebung mehr erfolgen kann und so eine genaue Positionierung des Verweilteils über den vorgestochenen Kanal garantiert ist.

In weiterer Ausgestaltung können das Stechteil und das Verweilteil in einem Gehäuse aufgenommen sein, in dem die Führung und insbesondere auch der Anschlag untergebracht ist/sind, wobei das Gehäuse einen Durchgang für das Stechteil und das Verweilteil aufweist. Das Gehäuse kann so angepasst werden, dass es die Handhabung der gesamten Vorrichtung erleichtert und insbesondere noch zusätzliche Elemente aufnimmt oder fixiert, die benötigt werden oder deren Einsatz besonderen Vorteil bringt.

An der Führung bzw. im Gehäuse kann bei einer bevorzugten Variante der erfindungsgemäßen Vorrichtung eine Stechteilaufnahme angeordnet sein, die das Stechteil nach der Entfernung aus der Stechposition aufnimmt und sicher verstaut. Das Stechteil wird bei der erfindungsgemäßen Vorrichtung nach dem Vorstechen aus seiner Position über dem vorgestochenen Kanal ausgerückt bzw. entfernt. Wenn es in die oben beschriebene Stechteilaufnahme eingeführt wird, ergeben sich die Vorteile, dass es einerseits nicht mehr entsorgt werden muss und andererseits keine Verletzungsrisiken für den Anwender mehr entstehen.

Als zusätzliches oder integriertes Bauteil kann gemäß einer vorteilhaften Ausführungsform der Erfindung an der Führung bzw. am Gehäuse ein Hautspanner angeordnet werden, der nach dem Stechen an der Haut um den Kanal herum angreift und den Kanal offen hält, so dass das Verweilteil noch einfacher eingebracht werden kann.

Die oben beschriebene Führung für Stechteil bzw. Verbleibteil kann jedwede Ausgestaltung annehmen. Sie kann eine Kurvenführung, eine Linearführung oder eine Kombination aus Kurven- und Linearführung sein. Sie ist mit Vorteil so ausgestaltet, dass die Einstechbewegung des Stechteils und die Setzbewegung des Verbleibteils unabhängig voneinander durchgeführt werden können, auch wenn die beiden genannten Teile ansonsten bewegungsgekoppelt sind.

In einer weiteren bevorzugten Ausführungsform der erfindungsgemässen Vorrichtung sind die Bewegung des Setzteils und des Verweilteils derart gekoppelt, dass die Rückzugsbewegung des Setzteil mit der Einführungsbewegung des Verweilteils teilweise überlappt. Bevorzugt besteht diese teilweise Überlappung darin, dass der Setzteil im wesentlichen vollständig aus der Haut zurückgezogen ist und nur noch dessen distale Spitze sich in der Haut befindet und mit der Einführbewegung des Verweilteils überlappt.

In weiterer, vorteilhafter Ausgestaltung der erfindungsgemäßen Vorrichtung ist das Verweilteil aus einem Material hergestellt, das bei Umgebungs- bzw. Zimmertemperatur eine größere Steifigkeit aufweist als bei Körpertemperatur. Es können beispielsweise Kunststoffe verwendet werden, die zu dem Zeitpunkt vor dem Einbringen des Verbleibteils relativ starr sind, um die Einbringung in die Haut zu erleichtern. Dies kann durch ein temperatursensitives Material erzielt werden, das bei Zimmertemperatur oder "normaler" Umgebungstemperatur (etwa 18 bis 24° C) relativ starr ist, in der Haut aber relativ weich wird, also bei Körper-Hauttemperatur.

Die Erfindung betrifft ferner ein Verfahren zum Setzen von Hautpenetratoren mit einem Stechteil, das die Haut durchsticht, und mit einem Verweilteil, das in eine vom Stechteil erzeugte Hautöffnung oder Hautkanal eingebracht wird und dort verbleibt. Beim erfindungsgemäBen Verfahren werden das Stechteil und das Verweilteil, die separat an einer Setzvorrichtung vorgesehen sind, mittels einer Führung nacheinander in die Haut gesetzt. In oder an der Führung werden das Verbleibteil und das Stechteil in die Stechposition verbracht.

Das Verfahren weist die schon vorab im Zusammenhang mit der erfindungsgemäßen Vorrichtung erzielbaren Vorteile auf. Natürlich kann bei der Durchführung des Verfahrens eine Vorrichtung verwendet werden, wie sie oben in verschiedenen Ausführungen beschrieben worden ist.

Der Begriff "Stechteil" wie er im Zusammenhang mit der vorliegenden Anmeldung verwendet wird, umfasst neben mechanischen Strukturen wie z.B. Nadeln, auch Flüssigkeitsstrahlen, welche zur Eröffnung der Haut dienen können, Laser und pyrotechnische Vorrichtungen.

Der Begriff "sequentielle Einführung" wie er im Zusammenhang mit der vorliegenden Anmeldung verwendet wird, umfasst neben einer verständigen zeitlichen Trennung der beiden Vorgänge (Einstechen des Einstechteils und Setzvorgang des Verweilteils) auch Vorgänge, bei welchen eine zeitliche Überlappung des Einstechvorgangs und des Setzvorgangs vorkommt d.h. die beiden Vorgänge sind phasenverschoben. Eine mögliche Überlappung besteht z.B. darin, dass der Verweilteil bereits in die Haut eingeführt wird während der Stechteil aus der Haut zurückgezogen wird. Eine weitere Überlappung besteht darin, dass der Einstechteil im wesentlichen vollständig aus der Haut zurückgezogen ist, wenn der Verweilteil in die Haut eingeführt wird. Die Bewegungen von Einstechteil und Verweilteil sind gekoppelt aber nicht identisch d.h. zeitlich versetzt.

Die Erfindung wird im Weiteren anhand bevorzugter Ausführungsformen näher erläutert. Sie kann alle hierin genannten Merkmale einzeln oder in jedweder Kombination aufweisen. In den Zeichnungen zeigen:
- Figur 1:: eine erste Ausführungsform der erfindungsgemässen Vorrichtung zum Setzen von Hautpenetratoren mit einem Rotationsmesser als Stechteil vor der Applikation;
- Figur 2:: die erfindungsgemässe Vorrichtung der Figur 1 nach deren Applikation;
- Figur 3:: eine zweite Ausführungsform der erfindungsgemässen Vorrichtung mit einem translatorisch geführten Stechteil vor der Applikation;
- Figur 4:: die Vorrichtung gemäss Figur 3 im Zustand nach deren Applikation;
- Figur 5:: Vorderansicht einer exemplarischen Antriebsvorrichtung zum Einführen des Stechteils einer erfindungsgemässen Vorrichtung in der Ausgangsposition vor dem Einführen des Einstechteils;
- Figur 6:: Rückansicht der Antriebsvorrichtung gemäss Figur 6;
- Figur 7:: Vorderansicht der Antriebsvorrichtung nach Fig. 5, wobei der Stechteil in die Haut eingeführt ist;
- Figur 8:: Rückansicht der Antriebsvorrichtung nach Figur 7;
- Figur 9:: Vorderansicht der Antriebsvorrichtung gemäss Figur 5, wobei sich das Antriebselement in der unteren Endposition befindet;
- Figur 10:: Rückansicht der Antriebsvorrichtung gemäss Figur 9;
- Figur 11:: Vorderansicht der Antriebsvorrichtung gemäss Figur 5, wobei sich das Einstechteil in der oberen Endposition nach dem Rückzug aus der Haut befindet;
- Figur 12:: Rückansicht der Vorrichtung nach Figur 11;
- Figur 13:: Vorderansicht einer erfindungsgemässen Vorrichtung zum Einführen von Hautpenetratoren mit einem durch eine kurvenförmige Bahn geführten Einstechteil;
- Figur 14:: eine Aufsicht der Vorrichtung gemäss Figur 13;
- Figur 15:: eine zweiteilige Vorrichtung zum Einführen eines Verweilteils in Gewebe und
- Figur 16:: eine dreidimensionale Darstellung einer weiteren sequentiellen Vorrichtung zum Einführen von Hautpenetratoren.

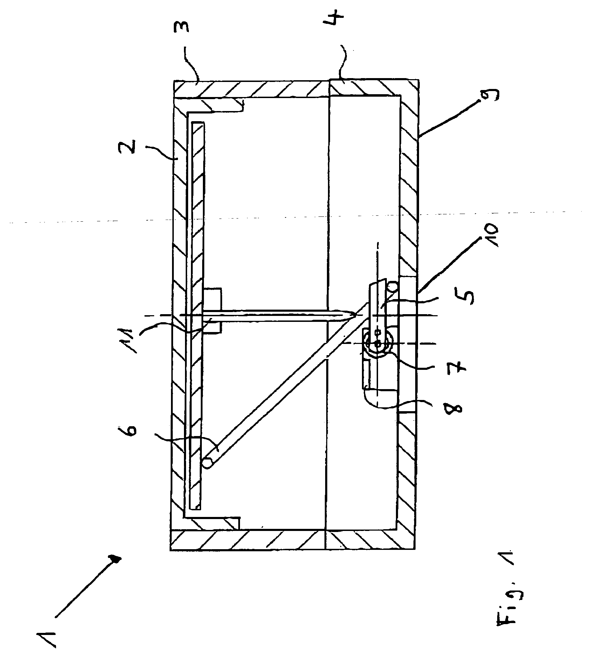

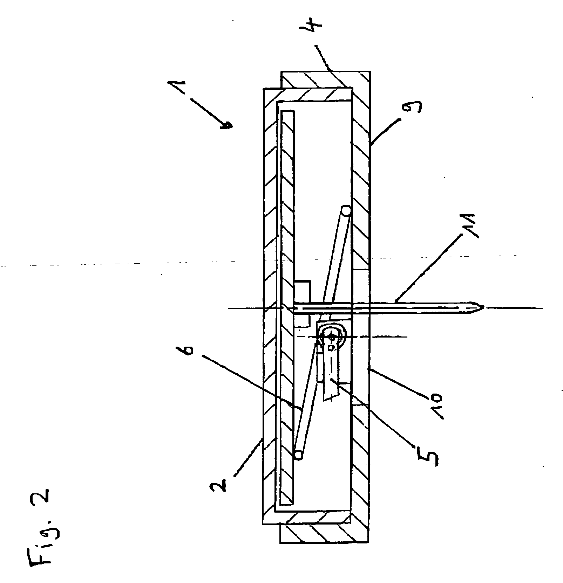

In den Figuren 1 und 2 ist eine erste Ausführungsform der erfindungsgemässen Vorrichtung zum Einführen von Hautpenetratoren dargestellt. Die Figur 1 zeigt die Vorrichtung 1 vor der Applikation d.h. vor dem Einführen des Hautpenetrators in oder durch die Haut und die Figur 2 nach erfolgter Einführung des Hautpenetrators in das subkutane Gewebe.

Der Gehäuseoberteil 2 ist durch einen Aufsatzring 3 mit dem Gehäuseunterteil 4 verbunden. Als Einstechteil dient in diesem Beispiel ein Rotationsmesser 5 als Einstechteil. Vor der Anwendung wird das Rotationsmesser 5 durch einen Bügel 6 in der Ausgangsstellung gehalten. Eine Feder 7 dient als Mittel zum Antreiben des Rotationsmessers 5 und ist in der dargestellten Ausgangsposition gespannt. Die Unterseite 9 des Gehäuses 4, welche auf einer Hautoberfläche positioniert wird, weist im Bereich des Rotationsmessers 5 eine Öffnung 10 auf, damit eine scharfe Kante des Rotationsmesser 5 aus der Gehäuseunterseite 9 hervortritt und die Haut durchdringen bzw. eröffnen kann. Durch dieselbe Öffnung 10 wird der Verweilteil 11 in die Haut eingeführt.

Das Rotationsmesser 5 ist derart in der Vorrichtung angeordnet, dass es bei Applikation der Vorrichtung einen kleinen Schnitt in den oberen Schichten der Haut verursacht, der ausreicht, um den Verweilteil 11 durch die Haut hindurch im subkutanen Gewebe eines Patienten zu platzieren.
Die Einführung des Hautpenetrators mittels der Vorrichtung der Figuren 1 und 2 läuft folgendermassen ab:
Auf den Gehäuseoberteil 2 wird eine nach unten gerichtete Kraft ausgeübt, welche diesen in Richtung Haut verschiebt, wobei der Gehäuseoberteil 2 vom Aufsatzring 3 gleitgeführt wird. Diese nach unten d.h. in Richtung Haut gerichtete Bewegung, des Gehäuseoberteil.s 2 führt zu einer seitlichen Verschiebung des Bügels 6 und zur Drehung des Rotationsmessers 5. Eine scharfe Kante des Rotationsmessers 5 dreht sich durch die Öffnung 10 aus dem Gehäuseunterteil 4 raus und verursacht einen Schnitt in den oberen Hautschichten und dreht sich bis zum Anschlag 8 zurück ins Gehäuse in seine Endposition.

Der Verweilteil 11 z.B. ein Sensor zur Messung der Glucosekonzentiation im interstitiellen Gewebe, wird, nachdem das Rotationsmesser 5 einen Hautschnitt gemacht hat, durch die Öffnung 10 des Gehäuseuntezteils 4 in das subkutane Gewebe eingeführt. Dies geschieht durch weiteres Verschieben des Gehäuseoberteil 2 in Richtung Haut bis dieser seine Endposition erreicht hat. In der Endposition laufen der Gehäuseoberteil 2 und der Gehäuseunterteil 4 ineinander und bilden bevorzugt eine kraftschlüssige Verbindung. Der Aufsatzning 3 kann dann von der Vorrichtung entfernt werden oder fällt ab.

Der Verweilteil 11 ist derart in der Vorrichtung angeordnet, dass ein genaues Einführen durch den mit dem Rotationsmesser 5 gemachten Schnitt durchführbar ist. Diese Anordnung des Verweilteils 11 in der Vorrichtung hat den Vorteil, dass nur ein kleiner Schnitt gemacht werden muss d.h. die erfindungsgemässe Vorrichtung erlaubt ein schonendes Einführen von z.B. Sensoren in die Haut.

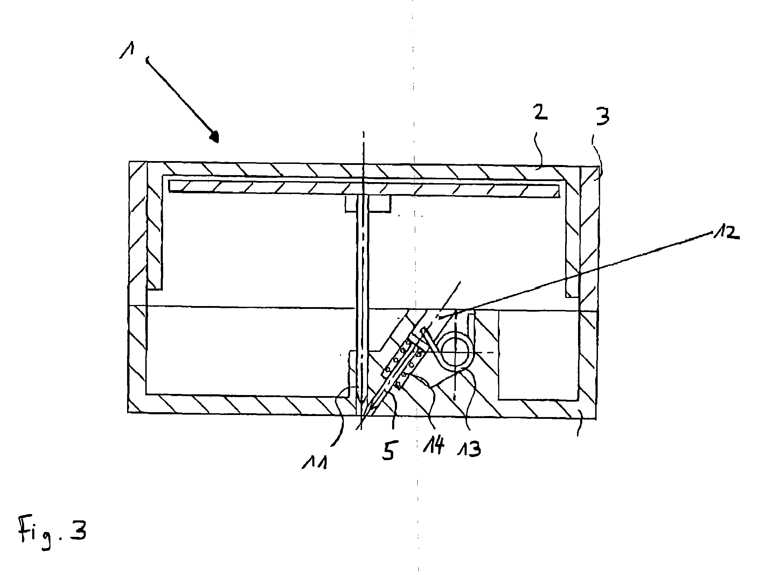

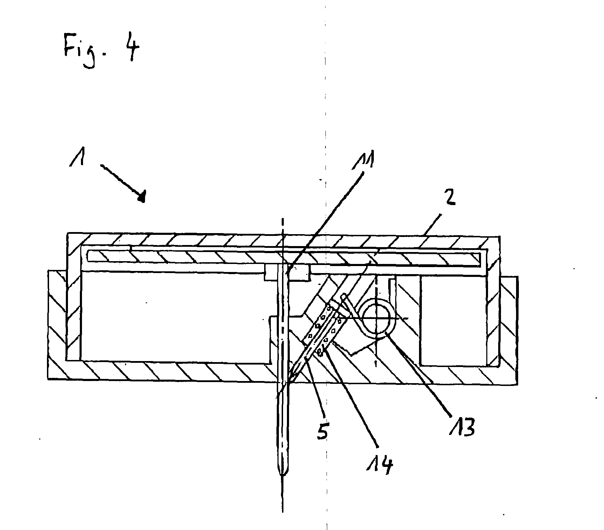

Die Figur 3 zeigt eine zweite Ausführungsform der erfindungsgemässen Vorrichtung vor deren Gebrauch und die Figur 4 zeigt die Vorrichtung nach Einführung des Verweilteils in das subkutane Gewebe eines Menschen d.h. nach deren Verwendung.

Die zweite Ausführungsform der erfindungsgemässen Vorrichtung unterscheidet sich von der ersten Ausführungsform, die in den Figuren 1 und 2 dargestellt ist, durch die Ausgestaltung des Einstechteils 5, der in der zweiten Ausführungsform in einer Führung 12 linear verschiebbar angeordnet ist.

Der Einstechteil 5 wird in der Führung 12 z.B. durch einen Bolzen (nicht dargestellt) in der Ausgangsstellung gehalten. Eine Feder 13 dient als Antriebsmittel zum Verschieben des Einstechteils 5 in Richtung Hautoberfläche. Bei der Applikation der Vorrichtung wird der Gehäuseoberteil 2 in Richtung Haut d.h. nach unten verschoben. Diese Abwärtsbewegung entsperrt den Bolzen und der Einstechteil 5 wird durch die gespannte Feder 13 in die Haut eingeführt. Das Einstechteil 5 wird durch die Feder 13 soweit nach vorne gedrückt bis die Feder 13 neben dem Einstechteil 5 vorbei in die Endstellung geht. In diesem Moment wird die Rückhaltefeder 14 wirksam und drückt den Einstechteil 5 in die Ausgangslage zurück. Der Aufsatzring 3 kann nach erfolgter Applikation der Vorrichtung abgenommen werden oder er fällt nach erfolgter Applikation selbständig ab.

Anstelle der Feder 13 als Antriebsmittel zum Einführen des Einstechteils 5 kann z.B. auch Gasdruck oder ein Elastomer als Antriebsmittel verwendet werden.

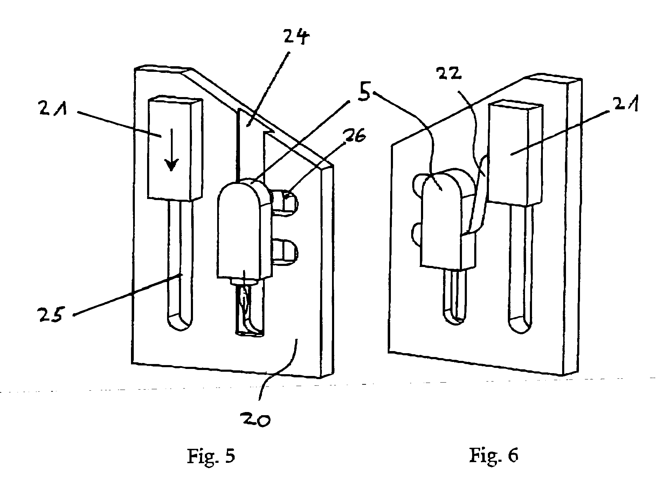

In den Figuren 5 bis 11 ist eine Antriebsvorrichtung zum Einführen des Stechteils einer exfindungsgemässen Vorrichtung dargestellt. In diesen Zeichnungen ist der Verweilteil, der in die Haut eingeführt wird, nicht dargestellt. Die Figuren 5, 7, 9 und 11 zeigen eine Vorderansicht der Vorrichtung und die Figuren 6, 8, 10 und 12 eine Rückansicht, wobei benachbarte Figuren denselben Zustand der Vorrichtung darstellen.

Die Antriebsvorrichtung umfasst eine Kulissenplatte 20, ein Antriebselement 21 und ein Einstechteil. 5. Das Antriebselement 21 ist mittels eines Federblechs 22 mit dem Einstechteil 5 verbunden, welches in einer F-förmigen Kulisse 24 geführt wird. Die Kulisse 24 zeichnet sich durch eine Stechteilaufnahme 26 aus, in welche das Einstechteil 5 nach durchgeführtem Einstechvorgang geführt wird. Das Antriebselement 21 kann durch eine vorgespannte Zug- oder Druckfeder oder ein Elastomerband angetrieben werden und wird in der Kulisse 25 geführt.

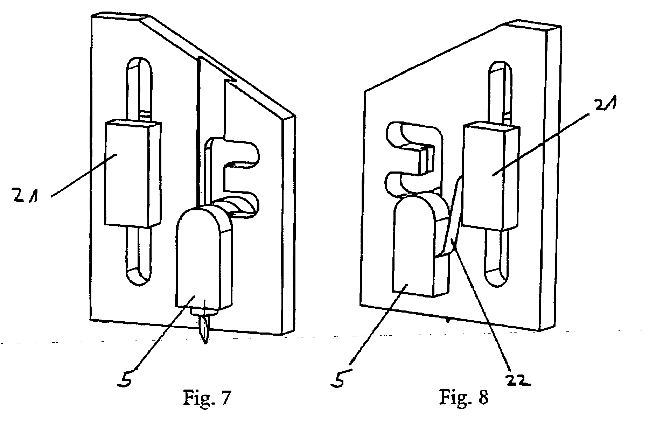

Bezugnehmend auf die Figuren 7 bis 12 lässt sich der Mechanismus der Antriebsvorrichtung wie folgt beschreiben:
Das Antriebselement 21 bewegt sich in der Kulisse 25 nach unten d.h. in Richtung Hautoberfläche. Das Einstechteil 5 wird über das Federblech 22 in Richtung Haut mitgenommen und durchsticht in der unteren Endposition die Hautoberfläche (Figuren 7 und 8).

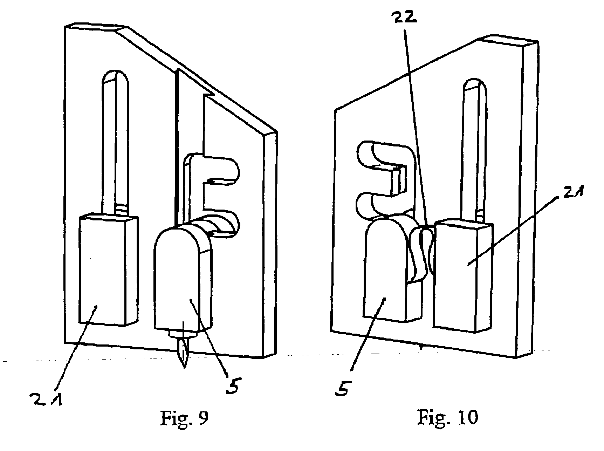

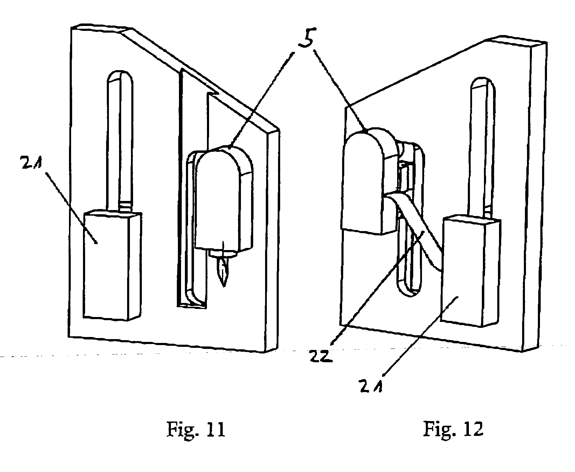

Das Antriebselement 21 bewegt sich weiter in Richtung Hautoberfläche und erreicht seine untere Endposition (Figuren 9 und 10). Aus der Figur 10 ist ersichtlich, dass in der unteren Endposition des Antriebselements 21 und des Einstechteils 5 sich das Federblech 22 deformiert hat d.h. das Federblech ist gespannt. Durch die Spannung des Federblechs 22 wird das Einstechteil 5 aus seiner unteren Endposition in der Kulisse 24 nach oben geschoben und entweicht seitlich in seine obere Endposition (Figuren 11 und 12).

Das seitliche entweichen des Einstechteils 5 gibt die Kulisse 24 frei, in welcher dann ein Verweilteil z.B. ein Sensor, durch die vom Einstechteil 5 geschaffene Hautöffnung in die Haut eingeführt werden kann. Die Bewegung des Einstechteils 5 kann so mit der Bewegung des Verweilteils gekoppelt sein, dass sobald der Einstechteil 5 seine obere Endposition erreicht hat, die Bewegung des Verweilteils ausgelöst wird.

Das in den Figuren 5 bis 11 dargestellte Federblech 22 kann z.B. durch eine Schenkelfeder ersetzt werden.

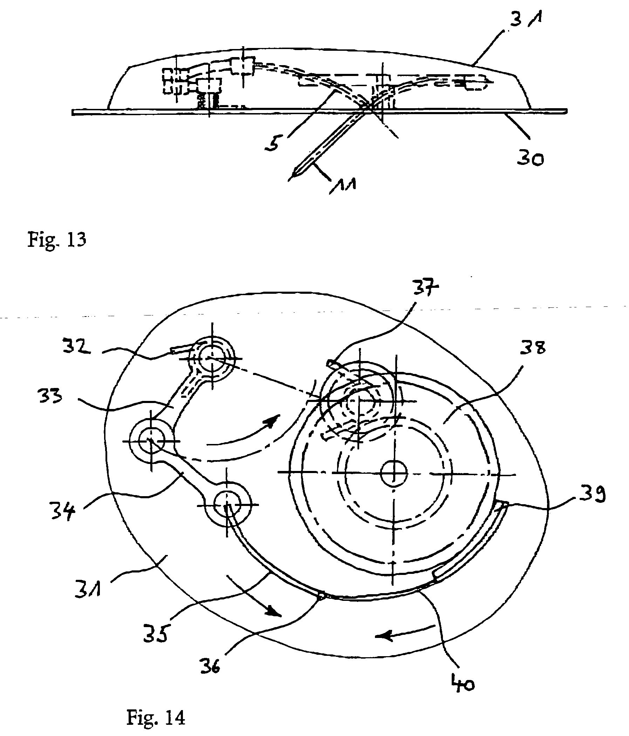

Die Figur 13 stellt eine Vorderansicht einer weiteren Ausführungsform der vorliegenden Erfindung dar und die Figur 14 zeigt eine Aufsicht der Vorrichtung. Die Vorrichtung ist über ein Pflaster 30 auf der Haut eines Patienten positioniert.

Die Vorrichtung umfasst ein Gehäuse 31, ein Einstechteil 5, einen Verweilteil 11 und zwei miteinander gekoppelte Mechanismen, wobei der erste Mechanismus dem Antrieb des Einstechteils 5 dient d.h. der erste Mechanismus ermöglicht die Eröffnung der Hautoberfläche mittels des Einstechteils 5. Der zweite Mechanismus steuert die Einführung des Verweilteils 11 ins subkutane Gewebe.

Das Einstechteil 5 wird über eine vorgespannte Schenkelfeder 32, die über zwei Kurbelarme 33 und 34 angetrieben ist, auf einer im Gehäuse integrierten Kurvenbahn 35 in die Haut eingeführt und nach erfolgter Einführung ins Gehäuse zurückgezogen. Nach dem Rückzug des Einstechteils 5 ins Gehäuse 31 verbleibt in der Haut eine kleine Öffnung 36. Das Verweilteil 11, das durch eine vorgespannte Schenkelfeder 37 und ein Zahnradgetriebe 38 angetrieben wird, wird mittels einem Führungselement 39 und der im Gehäuse integrierten Kurvenbahn 40 durch die vom Einstechteil 5 geschaffene Hautöffnung 36 ins subkutane Gewebe eingeführt.

Der Einstechvorgang kann manuell vom Anwender über z.B. einen Betätigungsknopf (nicht dargestellt) ausgelöst werden. Der nachfolgende Applikationsvorgang zur Einführung des Verweilteils 11 wird durch die Kurbelarme 33 und 34 ausgelöst d.h. die Einführung des Einstechteils und des Verweilteils sind miteinander derart gekoppelt, dass nach Rückzug des Einstcchteils 5 die Einführung des Verweilteils 11 ausgelöst wird.

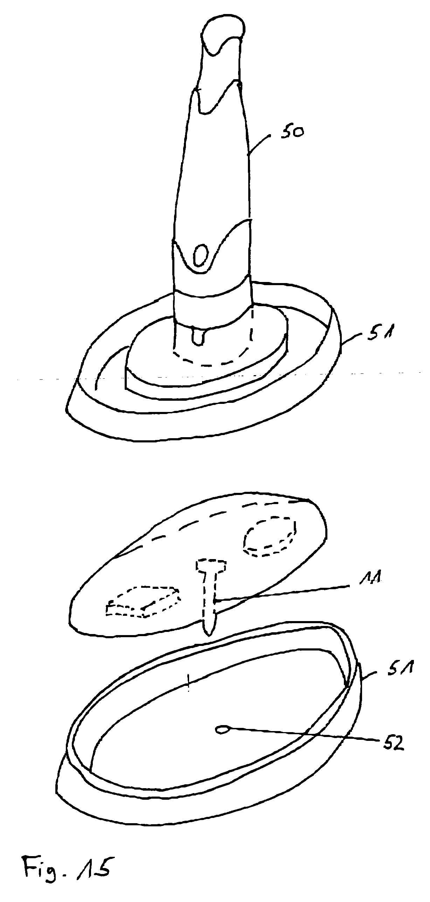

In der Figur 15 ist eine Ausführungsform der vorliegenden Erfindung dargestellt, bei welcher der Einstechteil und der Verweilteil nicht als Teile einer Vorrichtung vorgesehen sind, sondern als zwei separate Vorrichtungen ausgestaltet sind. Als Einstechvotrichtung kann z.B. eine herkömmliche Stcchhilfe wie Accu-Check Softelix oder Accu-Check Multiclix verwendet werden. Der Verweilteil. z.B. ein Sensor, kann in einer Vorrichtung integriert sein, welche z.B. die Elektronik zur Steuerung des Sensors, einen Datenspeicher und ein Telemetrie System zur Datenübermittlung an ein Drittgerät umfasst.

Bei der Ausführungsform mit zwei separaten Vorrichtungen ist das Verfahren zum Setzen eines z.B. subkutanen Sensors wie folgt:
Ein Patient bringt an der Hautstelle, an welcher er den Sensor setzen möchte, z.B. einen Positionierungsring 51 an. Der Positionierungsring 51 weist z.B. eine flächige Unterseite mit einer Öffnung 52 auf, durch welche der Einstechteil 5 der Binstechvorrichtung 50 eingeführt wird und eine umlaufende Mantelfläche. Damit der Positionsring 51 auf der Hautoberfläche haftet, weist er an seiner Unterseite z.B. ein Pflaster auf. Die Einstechvorrichtung 50 wird dann in den Ring eingesetzt und darin lösbar fixiert, um ein genaues Lochen der Haut zu ermöglichen. Die Fixierung der Einstechvorrichtung 50 kann z.B. über die Mantelfläche des Rings erfolgen.
   Der Patient führt dann den Stechvorgang durch und löst nach erfolgtem Stechvorgang die Einstechvorrichtung 50 vom Positionsring 51. Danach führt er z.B. den Sensor der Verweilvorrichtung durch die Hautöffnung ein und fixiert die Verweilvorrichtung am Positionsring. Nach Gebrauch wird die Verweilvorrichtung mit dem Positionsring von der Hautoberfläche entfernt und entsorgt.

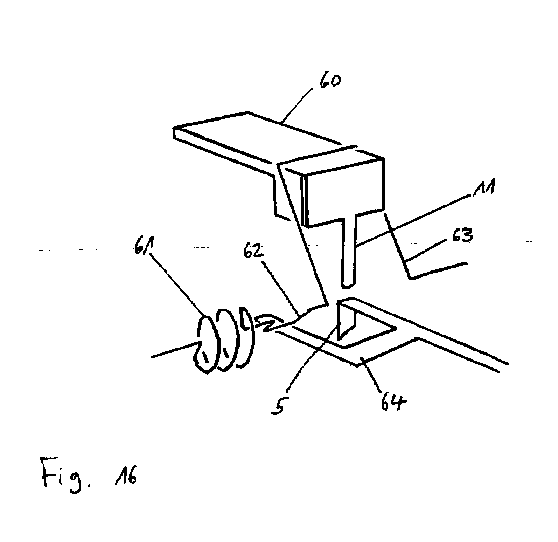

Figur 16 zeigt eine weitere beispielhafte Vorrichtung zur sequentiellen Einführung eines Verweilteils 11. Die Vorrichtung umfasst eine vertikal geführte Basisplatte 60, eine Antriebsvorrichtung 61, 62, 63 und ein Einstechteil 5.

Die Antriebsvorrichtung umfasst einen Federstahldraht, welcher an einer Stelle zu einer Spiralfeder 61 gebogen ist und an zwei Stellen vorstehende Bügel 62, 63 aufweist, welche nacheinander auf das Stechelement 5 und auf das Verweilteil 11 wirken.
Dabei sind die Bügel 62 und 63 um 90° gegeneinander versetzt, so dass beim Insertionsvorgang zuerst Bügel 62 über einen Bügel 64 das Stechelement 5 hinunterdrückt, wonach Bügel 63 über eine auf die Basisplatte 60 wirkende Kraft das Verweilteil 11 hinunterdrückt. Während des Insertionsvorgangs drehen sich die Bügel 62 und 63 um nahezu 180°. Durch den Winkelversatz wird erreicht, dass Bügel 62 in vertikaler Richtung eine Abwärtsbewegung gefolgt von einer Aufwärtsbewegung ausführt, während Bügel 63 sich in vertikaler Richtung ausschliesslich nach unten bewegt.

Der beschriebene Ablauf kann auch über zwei versetzte Nockenscheiben die an einen Antrieb gekoppelt werden realisiert werden
In einer alternativen Ausführung wirkt Bügel 63 nicht als Antrieb, sondern als Hemmung auf die Basisplatte 60, welche in diesem Fall durch einen externen Antrieb (nicht eingezeichnet) nach unten getrieben wird.

## Patentansprüche

1. Vorrichtung zum Setzen von Hautpenetratoren (1) mit einem Stechteil (5), das die Haut durchsticht, und mit einem Verweilteil (11), der durch eine vom Stechteil (5) erzeugte Öffnung in die Haut eingebracht wird und dort verbleibt, **dadurch gekennzeichnet, dass** das Stechteil (5) und Verweilteil (11) separat an der Setzvorrichtung (1) vorgesehen sind, und durch mindestens eine Führung (12, 24, 35, 39) in oder an welcher das Verweilteil (11) nach dem Stechteil (5) in dessen Stechposition verbracht wird, um dort in die Haut gesetzt zu werden oder der Stechteil (5) und der Verweilteil (11) sind als separate Vorrichtungen ausgestaltet.

2. Vorrichtung nach Anspruch 1, **dadurch gekennzeichnet, dass** das Stechteil (5) und der Verweilteil (11) in oder an einem Gehäuse angeordnet sind.

3. Vorrichtung nach Anspruch 2, **dadurch gekennzeichnet, dass** das Stechteil (5) und der Verweilteil (11) durch eine Führung (24) in die Haut eingeführt werden.

4. Vorrichtung nach Anspruch 3, **dadurch gekennzeichnet, dass** die Führung (24) einen Haltepunkt aufweist, welcher der Stech- bzw. Setzposition zugeordnet ist.

5. Vorrichtung nach Anspruch 4, **dadurch gekennzeichnet, dass** der Haltepunkt durch einen Anschlag für den Stechteil (5) und/oder den Verweilteil (11) gebildet wird.

6. Vorrichtung nach Anspruch 5, **dadurch gekennzeichnet, dass** der Stechteil (5) und der Verweilteil (11) bewegungsgekoppelt in der Führung (24) geführt werden und der Haltepunkt durch einen Anschlag erzeugt wird, an den der Stechteil (5) nach dem Stechen herangeführt wird, wobei er den Verweilteil (11) in die Setzposition mitnimmt.

7. Vorrichtung nach Anspruch 4, **dadurch gekennzeichnet, dass** der Haltepunkt durch einen Rasteingriff für den Stechteil (5) und/oder den Verweilteil (11) gebildet wird, wobei der Rasteingriff in oder an der Führung (24) angeordnet ist.

8. Vorrichtung nach Anspruch 7, **dadurch gekennzeichnet, dass** ein einziger wieder entrastbarer Rasteingriff in oder an der Führung (24) vorgesehen ist, in den zunächst der Stechteil (5) und danach der Verweilteil (11) einrasten kann.

9. Vorrichtung nach einem der Ansprüche 3 bis 8, **dadurch gekennzeichnet, dass** ein Rückhalteglied an oder in der Führung (24) vorhanden ist, welches das Stechteil (5) in der ausgerückten Position hält oder die gekoppelte Einheit aus separatem Stechteil (5) und Verweilteil (11) in der Setzposition des Verweilteils hält.

10. Vorrichtung nach einem der Ansprüche 3 bis 9, **dadurch gekennzeichnet, dass** an der Führung (24) bzw. im Gehäuse eine Stechteilaufnahme (26) angeordnet ist, die das Stechteil. (5) nach der Entfernung aus der Stechposition aufnimmt und sicher verstaut.

11. Vorrichtung nach Anspruch 1 oder 2, **dadurch gekennzeichnet, dass** das Stechteil (5) und der Verweilteil (11) durch unterschiedliche Führungen (12, 35, 40) geführt werden.

12. Vorrichtung nach einem der Ansprüche 5 bis 11, **dadurch gekennzeichnet, dass** das Stechteil (5) und das Verweilteil (11) in einem Gehäuse aufgenommen sind, in dem die Führung (12, 24, 35, 40) und insbesondere auch der Anschlag untergebracht ist/sind, wobei das Gehäuse einen Durchgang (10, 36) für das Stechteil (5) und das Verweilteil (11) aufweist.

13. Vorrichtung nach einem der Ansprüche 1 bis 12, **dadurch gekennzeichnet, dass** die Führung (12, 24, 35, 40) eine Kurvenführung, eine Linearführung oder eine Kombination aus Kurven- und Linearführung ist.

14. Vorrichtung nach einem der Ansprüche 1 bis 13, **dadurch gekennzeichnet, dass** das Verweilteil (11) aus einem Material hergestellt ist, das bei Umgebungs- bzw. Zimmertemperatur eine gröBere Steifigkeit aufweist als bei Körpertemperatur.

15. Vorrichtung nach einem der Ansprüche 1 bis 14, **dadurch gekennzeichnet, dass** es sich beim verweilleil (11) um einen Sensor zur Messung eines physiologischen Parameters handelt, bevorzugt um einen Sensor zur Messung der interstitiellen Glucosekonzentration.

16. Verfahren zum Setzen von Hautpenetratoren mit einem Stechteil (5), das die Haut durchsticht, und mit einem. Verweilteil (11), das durch eine vom Stechteil (5) erzeugte Hautöffnung eingebracht wird und dort verbleibt, bei dem das Stechteil (5) und das Verweilteil (11), die separat an einer Setzvorrichtung (1) vorgesehen sind, mittels mindestens einer Führung, in oder an welcher das Verbleibteil (11) und das Stechteil (5) in dessen Stechposition verbracht werden, nacheinander in die Haut gesetzt werden.

17. Verfahren nach Anspruch 16, bei dem eine Vorrichtung nach einem der Ansprüche 1 bis 15 zum Setzen eines Hautpenetrators verwendet wird.
